# EUROPÄISCHE PATENTSCHRIFT

(11) **EP 4 203 822 B1**
(45) Veröffentlichungstag und Bekanntmachung des Hinweises auf die Patenterteilung: **18.02.2026**
(21) Anmeldenummer: 21835308.4
(22) Anmeldetag: 13.12.2021
(51) Int. Cl.: A61B 17/80, A61B 17/56, A61B 90/00

(54) **FINGERFREIES MAXILLA-REPOSITIONSIMPLANTAT**
MAXILLA REPOSITIONING IMPLANT WITHOUT FINGER-TYPE STRUCTURES
IMPLANT DE REPOSITIONNEMENT DE MAXILLAIRE EXEMPT DE DOIGT

(30) Priorität: 18.12.2020 DE 102020134246
(43) Veröffentlichungstag der Anmeldung: 05.07.2023
(73) Patentinhaber: Karl Leibinger Asset Management GmbH & Co. KG, 78570 Mühlheim an der Donau (DE)
(72) Erfinder: REINAUER, Frank, 78576 Emmingen-Liptingen (DE); GABELE, Lorenz, 88605 Sauldorf (DE)
(74) Vertreter: Winter, Brandl - Partnerschaft mbB
(86) Internationale Anmeldenummer: PCT/EP2021/085558
(87) Internationale Veröffentlichungsnummer: WO 2022/128960

(56) Entgegenhaltungen:
- US-A1- 2011 144 698
- US-A1- 2012 277 749
- US-A1- 2017 156 770
- US-A1- 2018 344 464

## Beschreibung

Die Erfindung betrifft ein Maxilla-Repositionsimplantat zum dreidimensionalen exakten Ausrichten eines Maxilla-Verlagerungsabschnitts relativ zu einem schädelfesten Maxilla-Restabschnitt. Als Maxilla wird hier, wie üblich, der menschliche Oberkieferknochen bezeichnet. Ein Teil des Oberkieferknochens kann mittels eines chirurgischen Eingriffs vollständig vom Rest des Oberkiefers getrennt werden. Dadurch lässt sich ein Maxilla-Verlagerungsabschnitt von einem schädelfesten Maxilla-Restabschnitt entfernen. Auf diese Weise können Fehlstellungen im Kiefer behoben werden. Der Maxilla-Restabschnitt ist jedoch nach der Verlagerung wieder am Maxilla-Verlagerungsabschnitt zu befestigen. Dafür werden üblicherweise Maxilla-Repositionsimplantate eingesetzt.

Solche Maxilla-Repositionsimplantate sind aus diversen Druckschriften bereits bekannt, so etwa aus der EP 2 563 244 B1. Die US 2011 / 144 698 A1 offenbart ein Mandibula-Repositionsimplantat mit ansonsten ähnlichen Eigenschaften.

Dort ist ein präoperativ maßgefertigtes Implantat zur Verwendung in der orthognathen Chirurgie offenbart. Bei der orthognathen Chirurgie wird ein erster Teil eines Oberkiefers von einem zweiten Teil des Oberkiefers getrennt und im Rahmen dieses Verfahrens dann das Implantat eingesetzt. Das Implantat umfasst ein Plattenbauteil, das vorgeformt ist, zu einer präoperativen Form des Oberkiefers zu korrespondieren und mehrere nichtlineare Wellungen enthält, die zu bestimmten Oberflächenteilen des ersten Teils des Oberkiefers korrespondieren, wobei das Plattenbauteil zumindest eine Fixieröffnung definiert, dieses sich durch das Plattenbauteil hindurch erstreckt und eingerichtet ist, ein Knochenfixierelement aufzunehmen, um das Plattenbauteil am ersten Teil des Oberkiefers zu befestigen. Ferner sind eine Mehrzahl von (freien) Finger vorhanden, die sich weg von dem Plattenbauteil erstrecken, wobei die (freien) Finger vorgeformt sind, zur Form des zweiten Teils des Oberkiefers zu korrespondieren und mehrere nichtlineare Wellungen enthalten, die zu bestimmten Oberflächenteilen des zweiten Teils des Oberkiefers korrespondieren.

Benachbarte Dokumente offenbaren ähnliche Implantate, wie bspw. die EP 2 687 168 B1, die EP 2 698 122 B1, die EP 2 563 242 B1, die EP 3 566 663 A2, die EP 3 263 050 B1, die EP 2 952 145 B1, die EP 2 767 246 A1, die EP 2 398 411 B1, die FR 2 942 125 B1, die WO 2014 090 964 A2, die EP 2 931 143 A2, die FR 2 999 071 A1, die EP 2 906 129 B1 und die WO 2014 043 370 A1. Auch die US 2018 / 344 464 A1, die US 2012 / 277 749 A1 oder die US 2017 / 156 770 A1 offenbaren ähnliche Implantate.

Die bekannten Implantate haben jedoch immer Nachteile. Insbesondere sind diese Implantate schwierig zu fertigen. Auch ist häufig die Stabilität nicht ausreichend. Werden die Implantate am Knochen verbaut und wieder mit Weichteilmaterial, wie Haut und Bindegewebe bedeckt, so ergibt sich ein unästhetisches Äußeres, was noch dazu einen unangenehmen Tragekomfort für den Patienten nach sich zieht. Häufig gibt es dazu Komplikationen und medizinisch negative Nebenwirkungen.

Ausgehend von einem Maxilla-Repositionsimplantat mit nachfolgenden Merkmalen hat sich die Erfindung die Aufgabe gesetzt diese Nachteile zu beseitigen. Darüber hinaus soll ein kosteneffizienteres Maxilla Repositionsimplantat unter Beseitigung auch anderer Nachteile geschaffen werden.

Die Erfindung geht dabei von einem Maxilla-Repositionsimplantat aus, das eine Basis besitzt, die wenigstens eine Platte aufweist, in der Schraubenlöcher vorhanden sind, wobei ein Abschnitt des Materials der Platte als Rand je ein Schraubenloch definiert (d. h. teilweise oder vollständig umgibt), wobei das Schraubenloch zur Aufnahme einer Knochenschraube vorgesehen ist, um über die in das jeweilige Schraubenloch einzusetzende Knochenschraube eine Befestigung am schädelfesten Maxilla-Restabschnitt sicherzustellen, wobei das Maxilla-Repositionsimplantat darüber hinaus einen Anschlussabschnitt besitzt, der von der Basis über Verbindungsstege beabstandet ist, wobei der Anschlussabschnitt wenigstens eine Platte aufweist, in der ebenfalls Schraubenlöcher vorhanden sind und wobei ein Abschnitt des Materials der Platte als Rand je ein Schraubenloch definiert (d. h. teilweise oder auch vollständig das Schraubenloch umgibt), um über eine in das jeweilige Schraubenloch einzusetzende Knochenschraube eine Befestigung am Maxilla Verlagerungsabschnitt sicher zu stellen.

Die eingangs genannte Aufgabe wird erfindungsgemäß dadurch gelöst, dass sich von der Platte des Anschlussabschnitts (wenigstens oder nur / genau) zwei (distinkte / voneinander getrennte / zweiseitig angebundene) Verbindungsstege zum Rand eines (einzigen / bestimmten) Schraubenlochs in der Platte der Basis erstrecken.

Es wird durch ein solches Maxilla-Repositionsimplantat eine höhere Festigkeit bei spannungsoptimiertem Design möglich. Auch ist ein geringerer Materialeinsatz, als bei herkömmlichen Implantaten realisierbar. Eine gewebeverträglichere Ausgestaltung und die Beseitigung der eingangs genannten Nachteile ist die überraschende Folge. Eine besonders zur EP 2 563 244 B1 völlig andersartige Lösung mit unerwartet positiven Effekten stellt sich ein.

Es sei daran erinnert, dass sowohl die Basis, als auch der Anschlussabschnitt bzgl. ihrer der Maxilla zugewandten Seite an die patientenspezifische Geometrie des Knochens angepasst ist. Die Oberflächenkontur der Maxilla ist sowohl im Bereich des Maxilla-Verlagerungsabschnitts, als auch im Bereich des schädelfesten Maxilla-Restabschnitts, mittels MRT-, CRT- und/oder Röntgen-Einsatz erhoben und mit nachfolgenden CAD- / CAM-Fertigungsverfahren in eine identische Kontur der Platte der Basis für den schädelfesten Maxilla Restabschnitt und der Platte des Anschlussabschnittes für den Maxilla-Verlagerungsabschnitt kopiert. Dabei können üblicherweise Sinterverfahren, wie Laser-Sinterverfahren, etwa das selektive Laser-Sinterverfahren, eingesetzt werden.

Für den Schutz im Ausland, insbesondere in den Vereinigten Staaten von Amerika sei darauf hingewiesen, dass die Erfindung auch ein Verfahren zum Einsetzen des Maxilla-Repositionsimplantates in/an den Patienten umfasst.

Ein Verfahren zum Fertigen des Maxilla-Repositionsimplantates wird ebenfalls beschrieben, das keinen Teil der beanspruchten Erfindung bildet. Es dient lediglich dem Verständnis der Erfindung.

Vorteilhafte Ausführungsformen sind in den Unteransprüchen beansprucht und werden nachfolgend näher erläutert. Dabei ist es auch denkbar, dass diese Aspekte unabhängig davon weiter verfolgt werden, dass sich von der Platte des Anschlussabschnittes (wenigstens oder nur / genau) zwei Verbindungsstege zum Rand eines (einzigen / bestimmten) Schraubenloches in der Platte der Basis erstrecken. Allerdings ist es selbstverständlich bevorzugt, wenn diese Merkmalskombination auch bei den Ausführungsformen der Unteransprüche realisiert ist.

Um ein besonders gewebeschonendes Design - insbesondere zwischen Schraubenlochbefestigungspunkten am Knochen - zu realisieren, hat es sich bewährt, wenn jeder/der Verbindungssteg (vorzugsweise an wenigstens einer Stelle oder über die gesamte Länge des Verbindungssteges) einen elliptischen Querschnitt besitzt.

Für die Fertigung ist es dabei von Vorteil, wenn der elliptische Querschnitt an wenigstens einer Stelle oder über die gesamte Länge des Verbindungssteges gesehen, die spezielle Form eines Kreises einnimmt. Dabei ist es auch möglich, dass sich Abflachungen auf der maxilla-abgewandten Oberseite des Verbindungssteges zeigen und/oder der elliptische Querschnitt über die Länge gesehen variiert. Auch können Erhöhungen oder Vertiefungen, insbesondere Krümmungen eingebracht sein. Eine besonders ästhetische Erscheinung bei Bedeckung des Implantats bzw. der Implantatsabschnitte mit Weichgewebe ist nach dem Einsetzen des Implantats die vorteilhafte Folge.

Der Stabilität ist es zuträglich, wenn die Platte der Basis und/oder die Platte des Anschlussabschnitts wenigstens eine Stelle besitzt, die eine größere Dicke als der Verbindungssteg (insbesondere an seiner dicksten Stelle) aufweist. In diesem Zusammenhang ist auch wünschenswert, dass die Verbindungsstege distinkt von den Bestandteilen der Platte ausgebildet sind und/oder die Fläche des Querschnitts des Verbindungssteges zwischen 70 % oder 80% und 120% (vorzugsweise 75% oder 100%) der Fläche des Schraubenloches beträgt oder die Breite des Verbindungssteges ungefähr dem Durchmesser eines der Schraubenlöcher entspricht.

Der Präzision der Ausrichtung des Maxilla Verlagerungsabschnitts relativ zum schädelfesten Maxilla Restabschnitt ist es zuträglich, wenn jede Platte der Basis und jede Platte des Anschlussabschnitts über genau zwei Verbindungsstege miteinander verbunden sind. Ungewollte Belastungen nach Art von Verbiegungen und Verdrehungen, insbesondere bei einer schlecht vermeidbaren Torsion der Basis relativ zum Anschlussabschnitt während des Einsetzens des Implantats, führen dann nicht zu einer Fehlstellung der beiden Maxilla-Abschnitte. Ein besonders gutes medizinisches Ergebnis kann dadurch sichergestellt werden.

Wenn die Verbindungsstege vollständig oder überwiegend parallel zueinander verlaufen, ist ein besonders spannungsoptimiertes Design realisierbar. Die Kräfte werden dann gleichartig abgeleitet.

Eine vorteilhafte Ausführungsform ist jedoch darüber hinaus dadurch gekennzeichnet, dass jeder Verbindungssteg ein erstes Ende und ein zweites Ende besitzt, wobei das erste Ende einmaterialig (integral / einheitlich) in den Rand eines ersten Schraubenloches der basisseitigen Platte übergeht und das zweite Ende einmaterialig (integral / einheitlich) in den Rand eines ersten Schraubenloches der anschlussabschlussseitigen Platte übergeht. Eine solch stufenlose Geometrie, die für den Patienten Entzündungen vermeidet, lässt sich dann realisieren. Darüber hinaus wird eine hohe Belastbarkeit des Implantats gewährleistet.

Im Zentrum der Erfindung steht ferner eine Ausführungsform, die dadurch gekennzeichnet ist, dass die basisseitige Platte über einen ersten Verbindungssteg und einen zweiten Verbindungssteg mit der anschlussseitigen Platte verbunden ist, wobei der erste Verbindungssteg und der zweite Verbindungssteg mit ihren ersten Enden an dem Rand eines einzigen ersten Schraubenlochs der basisseitigen Platte angreifen und a) entweder mit ihren zweiten Enden gemeinsam an dem Rand eines einzigen ersten Schraubenloches der anschlussabschnittseitigen Platte angreifen / daran befestigt sind / darin integriert sind oder b) der eine Verbindungssteg mit seinem zweiten Ende an dem Rand des ersten Schraubenlochs der anschlussabschnittseitigen Platte angreift / integriert ist und der andere Verbindungssteg an dem Rand eines zweiten Schraubenlochs der anschlussabschnittseitigen Platte angreift / integriert ist. Diese Ausführungsform bietet somit zwei Möglichkeiten realisiert zu werden, nämlich in einer besonders filigranen Variante (b) oder einer besonders stabilen Variante (a). Auch lassen sich dadurch geometrischen Besonderheiten auf der Oberfläche der Maxilla des speziellen, jeweils betroffenen Patienten Rechnung tragen. Besonders gute patientenspezifische Lösungen sind dann die Folge.

Dabei hat es sich bewährt, wenn der zweite Verbindungssteg sich über die Länge von der basisseitigen Platte aus gesehen zunehmend von dem ersten Verbindungssteg entfernt (von wenigstens nahe des ersten Endes in Richtung nahe des zweiten Endes) und in einer Weiterbildung in einem dem zweiten Ende nahen Bericht dem ersten Verbindungssteg wieder näher kommt). Eine Materialaufdickung bedingt durch die Nähe der beiden Verbindungsstege kann dadurch verhindert werden. Zusätzlich lässt sie die Stabilität erhöhen.

Ein geringer Materialeinsatz unter Erfüllung der Stabilitätserfordernisse lässt sich dann realisieren, wenn jede Platte genau 2 oder mehr, aber nicht mehr als 3, 4, 5 oder 6 Schraubenlöcher besitzt.

Ferner ist es von Vorteil, wenn alle Platten dieselbe Anzahl an Schraubenlöcher besitzt. Das Einsetzen des Implantates wird dadurch für den Operateur erleichtert.

Der Verträglichkeit des Implantats ist es zuträglich, wenn der Rand als Wulst ausgebildet ist. Vorteilhaft lässt sich dieser Wulst weiter bilden, wenn er auf seiner zum Schraubenloch zugewandten Seite (eher) konkav ausgebildet ist. Die Schraube kann mit ihrem Kopf dann bündig mit der Maxilla-abgewandten Oberfläche des Implantates abschließen und wackelfrei, d.h. präzise am Implantat anliegen. Die Verträglichkeit des Implantats wird bei eingesetzten Knochenschrauben dabei deutlich erhöht.

Dieser Effekt lässt sich noch steigern, wenn der Wulst auf seiner dem Schraubenloch abgewandten Seite konvex oder zylindrisch ausgebildet ist.

Für die Fertigung ist es von Vorteil, wenn jeder Verbindungssteg wenigstens einen in Längsrichtung geraden / linearen / ungebogenen Abschnitt besitzt.

Ein solches Implantat lässt sich besonders gut an den spezifischen Patienten anpassen, wenn zwei in Längsrichtung gerade / lineare / ungebogene Abschnitte über einen (gekrümmten) Bogenabschnitt verbunden sind, wobei vorteilhafterweise der Bogenabschnitt vorbereitet ist, beabstandet zu einem "Le-Fort-1-Gebiet" dieses zu überbrücken. Letztlich wird dadurch ein "Offset" realisiert, der Unverträglichkeiten in diesem Le-Fort-1-Gebiet vermeidet.

Es hat sich dabei bewährt, wenn der gerade Abschnitt oder ein Krümmungsabschnitt des Verbindungsstegs das erste Ende oder das zweite Ende des Verbindungsstegs ausbildet.

Bei der Erprobung des elliptischen Querschnittes hat es sich darüber hinaus auch bewährt, wenn die Hauptachse der Ellipse zwischen 25 % bis 75 %, vorzugsweise 50 % (+/- 10 %), größer ist als die Nebenachse. Besonders stabile und einen guten Tragekomfort ermöglichende Lösungen stellen sich dann ein.

Wenn das Maxilla Repositionsimplantat so bemessen ist, dass es nur im Bereich der Ränder der Schraubenlöcher in Kontakt mit der Maxilla steht, so lässt sich eine präzise Ausrichtung der beiden hier betroffenen Maxilla Abschnitte (Maxilla Verlagerungsabschnitt und schädelfester Maxilla Restabschnitt) bei Vermeidung von ungewollten Druckstellen und Entzündungen realisieren.

Es ist darüber hinaus von Vorteil, wenn die Verbindungsstege voneinander (materialig) getrennt sind (d. h., dass die Verbindungsstege grundsätzlich separat voneinander sind) und nur mittels eines ersten Befestigungsbereichs an ihrem ersten Ende an der Basis mittels eines zweiten Befestigungsbereichs an ihrem zweiten Ende an dem Anschlussabschnitt angebracht sind. Das Verwenden unnötig vielen Materials lässt sich dann vermeiden.

Es ist zweckmäßig, wenn die Verbindungsstege einen ebenen Versatz einer das Schraubenloch an der Basis aufnehmenden gedachten ersten Ebene zu einer das Schraubenloch in dem Anschlussabschnitt aufnehmenden gedachten zweiten Ebene sicherstellen.

Es ist von Vorteil, wenn die Längsachse einer basisseitigen Platte die Längsachse eines oder zweier davon abgehender Verbindungsstege vorgibt, zu welcher die Längsachse der anschlussabschnittseitigen Platte, an welcher der besagte Verbindungssteg angreift oder die besagten Verbindungsstege angreifen, fluchtet oder quer dazu - vorzugsweise orthogonal - verläuft.

Für die Fertigung, aber auch die Verträglichkeit des Implantats, ist es positiv, wenn die Ränder / Wulste der Schraubenlöcher (alle) gleich / gleichmäßig bleiben und/oder gleich dick sind.

Wenn zwei Platten der Basis über einen Haltebügel miteinander verbunden sind, so kann die Manipulierbarkeit des Maxilla-Repositionsimplantates verbessert werden. Insbesondere lässt sich der Einsetzvorgang vereinfachen. So kann dann bspw. das Maxilla-Repositionsimplantat als ein einheitliches Bauteil -umfassend wenigstens eine, besser zwei, noch besser drei oder optimalerweise 4, 5 oder 6 Platten an der Basis sowie wenigstens eine Platte, besser zwei Platten, oder gar 3 oder 4 oder sogar 5 oder 6 Platten am Anschlussabschnitt - mittels eines Haltebügels, zweier Haltebügel im Bereich der Basis, einem Haltebügel im Bereich des Anschlussabschnittes oder sogar zweier oder dreier Halteabschnitte vom Operateur gegriffen werden und als ein integrales Bauteil am Knochen, nämlich im ersten Schritt am schädelfesten Maxilla-Restabschnitt und danach am Maxilla-Verlagerungsabschnitt befestigt werden oder vice versa. Während dieses Eingriffes ist die Trennung mittels schneidender / fräsender Maßnahmen an der Maxilla zwischen dem Maxilla Verlagerungsabschnitt und dem schädelfesten Maxilla Restabschnitt (vorzugsweise im "Le-Fort-1-Gebiet") durchzuführen.

Im Rahmen dieser Operation hat es sich als besonders vorteilhaft herausgestellt, wenn ein Haltebügel auf der Anschlussabschnittseite des Maxilla-Repositionsimplantates vorhanden ist, der von der einen Gesichtshälfte zur anderen Gesichtshälfte ragt und somit den linken und den rechten Teil miteinander verbindet.

Dabei ist es besonders von Vorteil, wenn zwei Platten des Anschlussabschnittes über einen Haltebügel miteinander verbunden sind.

Genauso ist von Vorteil, wenn der Haltebügel über eine endseitige Sollbruchstelle an der jeweiligen Platte angebunden ist, da er dann mit einfachen Maßnahmen, ohne schneidend tätig zu werden, kraftsparend aus dem Implantat herausbrechbar ist.

Beim Einsetzvorgang hat es sich als vorteilhaft herausgestellt, wenn der Haltebügel selber (auch) eine abgerundete Kontur besitzt.

Besonders vorteilhafte Ausführungsformen lassen sich realisieren, wenn der Haltebügel a) eine der Maxilla zugewandte plane Fläche besitzt und eine der Maxilla abgewandte konvexe Fläche besitzt oder b) einen elliptischen, vorzugsweise kreisrunden Abschnitt / Querschnitt besitzt.

Für das Herausbrechen der Haltebügel vom eingesetzten Implantat, also vom Lösen des Haltebügels von den jeweiligen Platten, ist es vorteilhaft, wenn vorzugsweise mittig des Haltebügels (wenigstens / genau) eine Öse eingearbeitet ist, deren Längsachse quer - vorzugsweise orthogonal - zur Längsachse des Haltebügels ausgerichtet ist. In bestimmten Fällen kann es von Vorteil sein, wenn die Längsachse der Öse fluchtend zur Längsachse des Haltebügels verläuft.

Bei der Haltebügelausgestaltung ist es besonders von Vorteil, wenn der Haltebügel von der Maxilla im am Knochen befestigten Zustand beabstandet bleibt. Auf diese Weise wird genug Freiraum vorgehalten, um im eingesetzten Zustand ein Herausbrechen zu gewährleisten und eine Traumatisierung des Patienten im Haltebügelbereich zu vermeiden.

Wenn alle Platten des Anschlussabschnitts über mehrere Haltebügel miteinander verbunden sind, so lässt sich ein kompaktes Maxilla-Repositionsimplantat realisieren, das während der Operation präzise händelbar ist. Die Manipulierbarkeit während der Operation wird erleichtert.

Dabei ist es von Vorteil, wenn an einem einzigen Rand eines Schraubenloches zwei Verbindungsstege und a) ein Haltebügel oder b) zwei Haltebügel angreifen. Es kann auch bevorzugt sein, wenn c) an einer dieses Schraubenloch aufweisenden Platte ein Rand für ein weiteres / freies Schraubenloch ausgebildet ist, von welchem weder Verbindungsstege noch Haltebügel abgehen (es also verbindungssteg- und haltebügelfreie Ränder von Schraubenlöchern gibt), oder d) von jedem Rand eines Schraubenlochs entweder zwei Verbindungsstege oder wenigstens ein Haltebügel (besser zwei Haltebügel) abgeht / abgehen.

Es ist von Vorteil, wenn alle Platten über Verbindungsstege und/oder über Haltebügel miteinander verbunden sind. Ein einheitliches Maxilla Repositionsimplantat mit guter Handhabbarkeit ist dann die Folge.

Die Stabilität wird verbessert, wenn jede Platte nur Ränder für die Schraubenlöcher und darüber hinaus Ränder-verbindende und zu einer Platte mit geraden Enden ergänzende Füllbereiche besitzt. Die Füllbereiche sind dann zweckmäßigerweise überstandsfrei zu gestalten. Traumatisierungen sowie Entzündungen werden dadurch vermieden.

Darüber hinaus ist es von Vorteil, wenn zwei Verbindungsstege einen Leerraum umschließen.

Eine vorteilhafte Ausführungsform ist auch dadurch gekennzeichnet, dass zwischen den Rändern zweier Schraubenlöcher einer Platte und den daran anschließenden Füllbereichen ein Hohlraum vorhanden ist. Ein besonders leichtes und wenig Material einsetzendes Maxilla-Repositionsimplantat kann dann gestaltet werden, obwohl immer noch ausreichend Stabilität vorhanden ist.

Wenn zwei Platten und die sie verbindenden Verbindungsstege rautenartig aufgespannt sind, d. h. einen rautenartigen Leerraum definieren, so kann eine besonders hochbelastbare Maxilla-Repositionsimplantat-Ausgestaltung realisiert werden.

Erprobte Materialien lassen sich einsetzen, wenn das Implantat aus Metall, etwa einer Titan- oder Magnesiumlegierung aufgebaut ist oder aus Kunststoff aufgebaut ist, etwa PPA, PLLA oder PP. Gerade Magnesiumlegierungen haben jedoch unerwartete Vorteile.

Wünschenswert ist es natürlich, das Maxilla-Repositionsimplantat als patientenspezifisches Implantat auszubilden, vorzugsweise unter Einsatz von Sinterverfahren.

Das besagte Maxilla-Repositionsimplantat lässt sich gut an den Haltebügeln greifen. Die Platten und Verbindungsstege sind an die patientenspezifische Geometrie der Maxilla angepasst. Eine operationsabschließende Bedeckung mit Weichgewebe ist dabei auch vorausschauend berücksichtigt. Nach Eröffnung des Weichgewebes im Bereich der Maxilla während der Operation wird das Implantat an der Maxilla befestigt und entweder zuerst mittels Knochenschrauben am Maxilla-Verlagerungsabschnitt oder am schädelfesten Maxilla-Restabschnitt befestigt. Davor, währenddessen oder danach findet die Trennung dieser beiden Maxilla-Abschnitte statt. Nach Verlagerung des Maxilla-Verlagerungsabschnittes relativ zum ortsunveränderten und schädelfesten Maxilla-Restabschnittes, wird das Implantat mittels Knochenschrauben, die durch die noch freien Schraubenlöcher geführt werden, am bis zu diesem Zeitpunkt noch freien Maxilla-Abschnitt, also entweder dem schädelfesten Maxilla-Restabschnitt oder dem Maxilla-Verlagerungsabschnitt, befestigt. Eine präzise Endposition der beiden Maxilla-Abschnitte relativ zueinander und erfüllend die medizinischen Wunschvorstellungen, lässt sich dann realisieren. Bevor das Implantat wieder von Weichgewebe bedeckt wird, werden die Haltebügel entfernt, bspw. mittels eines Ausbrechens unter Nutzung der Schwächung im Bereich der Sollbruchstellen.

Die Erfindung wird nachfolgend auch mit Hilfe einer Zeichnung näher erläutert. Es zeigen:
- Fig. 1: eine erste Ausführungsform eines Maxilla-Repositionsimplantates ("double-ridge concept"), während des Einsetzens,
- Fig. 2: das Maxilla-Repositionsimplantat mit seinen über Verbindungsstege verbundenen Platten der Basis und des Anschlussabschnitts bei schon entfernten Haltebügeln in einer zu Fig. 1 vergleichbaren Ansicht,
- Fig. 3: eine gedrehte Darstellung auf das eingesetzte Maxilla-Repositionsimplantat im Zustand aus Fig. 2,
- Fig. 4: eine Vergrößerung des Bereichs IV aus Fig. 2 mit eingesetzten Knochenschrauben,
- Fig. 5: eine singuläre Darstellung der Seite eines einzigen Verbindungssteges aus Fig. 3,
- Fign. 6 und 7: Querschnitte entlang der Linien VI und VII durch den Verbindungssteg aus Fig. 5,
- Fig. 8: eine zweite Ausführungsform eines erfindungsgemäßen Maxilla-Repositionsimplantats ("double-ridge concept with optimized path"), wobei die beiden Verbindungsstege, welche eine Platte der Basis und eine Platte des Anschlussabschnitts miteinander verbinden, voneinander beabstandet sind, also gespreizt sind, ergo einen rautenartigen Leerraum definieren,
- Fig. 9: eine Darstellung des Maxilla-Repositionsimplantates aus Fig. 8 mit entfernten Haltebügeln,
- Fig. 10: eine Seitendarstellung einer Hälfte des Maxilla-Repositionsimplantates mit entfernten Haltebügeln zum Zustand, wie in Fig. 9 dargestellt,
- Fig. 11: eine Vergrößerung des Bereichs aus Fig. 9 mit eingesetzten Knochenschrauben,
- Fig. 12: eine seitliche Darstellung eines der Verbindungsstege aus Fig. 10, und
- Fign. 13 und 14: Querschnitte entlang den Linien XIII und XIV durch den jeweiligen Verbindungssteg aus Fig. 12.

Die Figuren sind lediglich schematischer Natur und dienen nur dem Verständnis der Erfindung. Gleiche Elemente sind mit denselben Bezugszeichen versehen.

Die erste Ausführungsform, die als "double-ridge concept" beschreiben wird, betrifft ein Maxilla-Repositionsimplantat 1. Das Maxilla-Repositionsimplantat greift mit einer Basis 2 an einem schädelfesten Maxilla-Restabschnitt 3 an. Ein Anschlussabschnitt 4 des Maxilla-Repositionsimplantats 1 greift an einem Maxilla-Verlagerungsabschnitt 5 an. Der schädelfeste Maxilla-Restabschnitt 3 und der Maxilla-Verlagerungsabschnitt 5 sind Teile der Maxilla, also des Oberkiefers.

Der Anschlussabschnitt 4 ist von der Basis 2 des Maxilla-Repositionsimplantates 1 durch Verbindungsstege 6 beabstandet. Dabei greifen die Verbindungsstege 6, von denen es in dem vorliegenden Ausführungsbeispiel acht Stück gibt, an Platten 7 an.

Vier Platten 7 gehören dabei zur Basis 2 und vier Platten 7 gehören dabei zum Anschlussabschnitt 4. Es können pro Basis 2 oder pro Anschlussabschnitt 4 zwei, drei, vier, fünf, sechs, sieben oder sogar acht Platten 7 verwendet sein. Im vorliegenden Ausführungsbeispiel gibt es gleichviele Platten 7 in der Basis 2 dem Anschlussabschnitt 4, nämlich jeweils vier. Die Zahlen können jedoch unterschiedlich sein.

Es gibt auch Haltebügel 8, die die Platten 7 der Basis 2 miteinander verbinden oder die Platten 7 des Anschlussabschnitts 4 miteinander verbinden. Letztlich sind die Haltebügel 8 so eingesetzt, dass wenigstens immer zwei Platten 7 der Basis 2 oder des Anschlussabschnitts 4 miteinander verbunden sind. Darüber hinaus gibt es einen zentralen Haltebügel 8, der zusätzlich mit dem Bezugszeichen 9 gekennzeichnet ist und welcher die Verbindung der linken Hälfte des Maxilla Repositionsimplantats 1 mit der rechten Hälfte des Maxilla Repositionsimplantats 1 im Bereich des Anschlussabschnitts 4 sicherstellt. Die Anordnung des zentralen Haltebügels 9 als Teil des Anschlussabschnittes 4 ist von Vorteil, da dann das Bauteil unterhalb der Nase angeordnet werden kann, was einen relativ guten Tragekomfort und ein gutes Erscheinungsbild realisiert.

Zurückkommend zu den Verbindungsstegen 6 ist bedeutsam, dass in vorzugsweise allen Platten 7 jeweils zwei Schraubenlöcher 10 vorhanden sind. Die Schraubenlöcher 10 werden durch das Material der Platten 7 gebildet. Das Material der Platten 7 definiert dabei einen Rand 11 für das jeweilige Schraubenloch 10. Der Rand 11 ist als Wulst 12 ausgebildet. Es sollte beachtet werden, dass jede Platte 7 mehr als zwei Schraubenlöcher 10 besitzen soll.

Im Übergangsbereich zwischen den Haltebügeln 8, also auch dem zentralen Haltebügel 9, zu den jeweiligen Platten 7 sind Ösen 13 vorgehalten. Die Ösen 13 weisen Längsachsen auf, die mit dem Bezugszeichen 14 versehen sind. Im Übergangsbereich zwischen den Haltebügeln 8 und den Platten 7 gibt es Sollbruchstellen 15. Diese sind exemplarisch an einem Haltebügel 8 dargestellt, gibt es jedoch an den meisten oder besser allen Haltebügeln 8.

Die Verbindungsstege 6 gehen immer von einer Platte 7 der Basis 2 ab, und stellen eine Verbindung mit dem Rand 11 einer Platte 7 des Anschlussabschnitts 4 sicher.

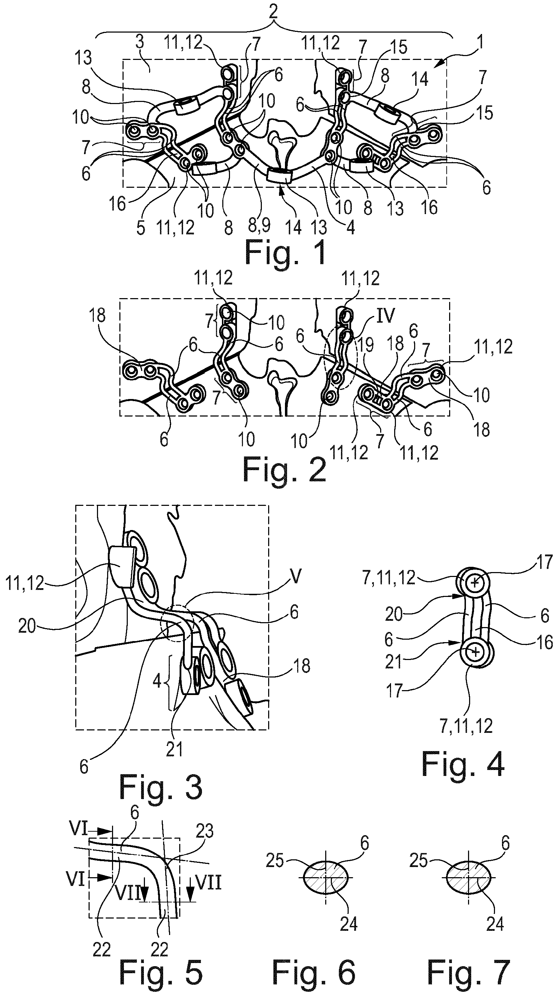

In dem Ausführungsbeispiel der Fig. 1 verlaufen die Verbindungsstege 6 der einen Platte 7 zur anderen Platte 7 zueinander (überwiegend) parallel, wobei deren erstes Ende im Rand 11 der einen Platte 7 im Rand 11 eines einzigen Schraubenloches enden und mit ihrem zweiten Ende im Rand 11 des Wulstes 12 eines einzigen Schraubenloches 10 der (anderen) Platte 7 des Anschlussabschnittes 4 münden. Mit anderen Worten verbinden beide Verbindungsstege 6 immer einen einzigen Rand eines einzigen Schraubenloches 10 der einen Platte 7 mit dem Rand 11 eines einzigen Schraubenloches 10 der anderen Platte 7. Die Verbindungsstege 6 treten immer paarweise auf. Zwischen den Verbindungsstegen 6 gibt es Leerräume 16.

Diese Leerräume sind auch gut in Fig. 4 zu erkennen, wobei dort ebenso die eingesetzten Knochenschrauben 17 angedeutet sind.

In Fig. 2 ist der haltebügelfreie Zustand, nach Beendigung der Operation dargestellt.

Die einzelnen Ränder 11 um die Schraubenlöcher 10 werden durch diese Schraubenlöcher 10 verbindende Füllbereiche 18 zur jeweiligen Platte 7 komplettiert. Es fällt auf, dass an wenigstens einer der Platten 7 des Anschlussabschnitts 4 jene die Ränder 11 zur Platte abschließenden Füllbereiche 18 einen Hohlraum 19 frei lassen.

Dass die Ränder 11 auf ihrer Außenseite zylindrisch ausgebildet sind, zumindest von der Außenkontur her, aber auf ihrer Innenseite eine konkave an einen Schraubenkopf angepasste Form besitzen, ist der Fig. 3 gut zu entnehmen.

In Fig. 5 ist angedeutet, dass wenigstens einer der Verbindungsstege 6, vorzugsweise alle Verbindungsstege 6, zwischen einem ersten Ende 20 des Verbindungsstegs 6 und einem zweiten Ende 21 des Verbindungsstegs 6 (siehe diesbezüglich Fig. 3) einen geraden Abschnitt 22 und wenigstens einen Bogenabschnitt 23 besitzen.

Dass der Querschnitt des Verbindungssteges 6, vorzugsweise aller Verbindungsstege 6 über die Länge gesehen gleichbleibt und von Verbindungssteg 6 zu Verbindungssteg 6 nicht variiert, ist in Zusammenschau der Fign. 5 bis 7 gut zu erkennen. Dabei ist eine Hauptachse 24 um den Faktor 11:8 größer als die Nebenachse 25.

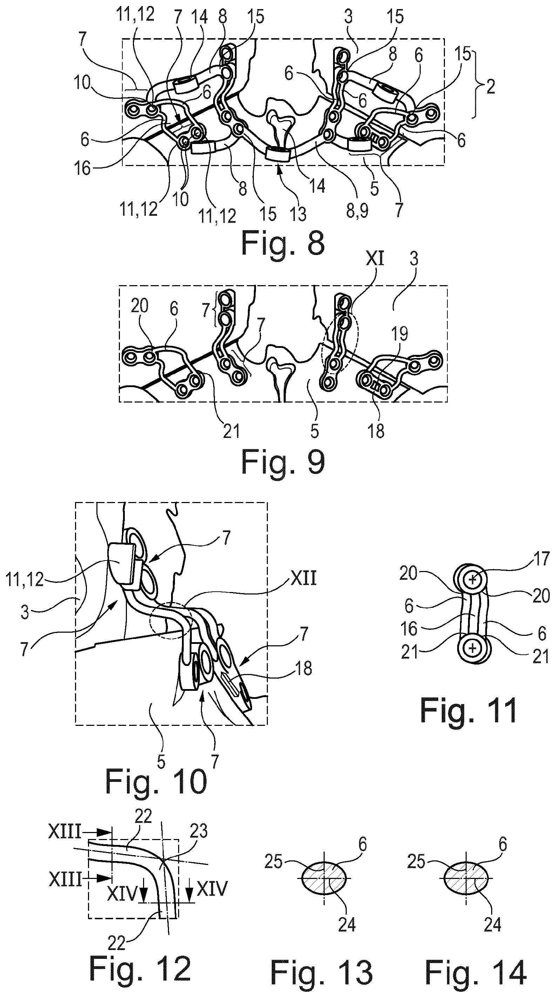

Die Ausführungsform, wie sie in den Fign. 8 bis 14 dargestellt ist und als "double-ridge concept with optimized path" bezeichnet ist, ist besonders durch die Vergrößerung der Leerräume 16 gekennzeichnet, derart, dass die Verbindungsstege 6, welche jeweils zwei Platten 7 verbinden, voneinander sich in Richtung des Anschlussabschnitts 4 voneinander tendenziell entfernen. Dabei geht der eine Verbindungssteg 6 vom Rand 11 einer basisseitigen Platte 7 um ein dortiges einziges Schraubenloch 10 zu einem Rand 11 um ein erstes Schraubenloch 10 in der Platte 7 des Anschlussabschnitts 4, wohingegen der andere Verbindungssteg 6, der vom selben Rand 11 um das Schraubenloch 10, von dem der erste Verbindungssteg 6 abgeht, zu einem anderen Rand 11 eines anderen Schraubenloches 10 derselben Platte 7 des Anschlussabschnitts 4 sich erstreckt, relativ zu dem ersten Verbindungssteg 6 gesehen. Der Leerraum 16 ist dann eher rautenförmig gestaltet. Der eine Verbindungssteg 6 und der andere Verbindungssteg 6 gehen dann zwar vom selben Rand 11 des Schraubenloches 10 der Basis 2 aus, enden aber an unterschiedlichen Rändern 11 von unterschiedlichen Schraubenlöchern 10 derselben Platte 7 des Anschlussabschnittes 4.

### Bezugszeichenliste

- 1: Maxilla Repositionsimplantat
- 2: Basis
- 3: schädelfester Maxilla Restabschnitt
- 4: Anschlussabschnitt
- 5: Maxilla Verlagerungsabschnitt
- 6: Verbindungssteg
- 7: Platte
- 8: Haltebügel
- 9: zentraler Haltebügel
- 10: Schraubenloch
- 11: Rand
- 12: Wulst
- 13: Öse
- 14: Längsachse
- 15: Sollbruchstelle
- 16: Leerraum
- 17: Knochenschraube
- 18: Füllbereich
- 19: Hohlraum
- 20: erstes Ende des Verbindungssteges
- 21: zweites Ende des Verbindungssteges
- 22: gerader Abschnitt des Verbindungssteges
- 23: Bogenabschnitt des Verbindungsstegs
- 24: Hauptachse
- 25: Nebenachse

## Patentansprüche

1. Maxilla-Repositionsimplantat (1), zum dreidimensionalen exakten Ausrichten eines Maxilla-Verlagerungsabschnitts (5) relativ zu einem schädelfesten Maxilla-Restabschnitt (3), wobei das Maxilla-Repositionsimplantat (1) eine Basis (3) besitzt, die wenigstens eine Platte (7) aufweist, in der Schraubenlöcher (10) vorhanden sind, wobei ein Abschnitt des Materials der Platte (7) als Rand (11) je ein Schraubenloch (10) definiert, das zur Aufnahme einer Knochenschraube (17) vorgesehen ist, um über die in das jeweilige Schraubenloch (10) einzusetzende Knochenschraube (17) eine Befestigung am schädelfesten Maxilla-Restabschnitt (3) sicher zu stellen, wobei das Maxilla-Repositionsimplantat (1) darüber hinaus einen Anschlussabschnitt (4) besitzt, der von der Basis (2) über Verbindungsstege (6) beabstandet ist, wobei der Anschlussabschnitt (4) wenigstens eine Platte (7) aufweist, in der Schraubenlöcher (10) vorhanden sind und wobei ein Abschnitt des Materials dieser Platte (7) als Rand (11) je ein Schraubenloch (10) definiert, um über eine in das jeweilige Schraubenloch (10) einzusetzende Knochenschraube (17) eine Befestigung am Maxilla-Verlagerungsabschnitt (5) sicher zu stellen, **dadurch gekennzeichnet, dass** sich von der Platte (7) des Anschlussabschnitts (4) zwei Verbindungsstege (6) zum Rand (11) eines Schraubenlochs (10) in der Platte (7) der Basis (2) erstrecken.

2. Maxilla-Repositionsimplantat (1) nach Anspruch 1, **dadurch gekennzeichnet, dass** jeder der Verbindungsstege (6) einen elliptischen Querschnitt besitzt.

3. Maxilla-Repositionsimplantat (1) nach Anspruch 2, **dadurch gekennzeichnet, dass** der elliptische Querschnitt die spezielle Form eines Kreises einnimmt.

4. Maxilla-Repositionsimplantat (1) nach einem der Ansprüche 1 bis 3, **dadurch gekennzeichnet, dass** die Platte (7) der Basis (2) und/oder die Platte (7) des Anschlussabschnitts (4) wenigstens eine Stelle besitzt/besitzen, die eine größere Dicke als der Verbindungssteg (6) aufweist/aufweisen.

5. Maxilla-Repositionsimplantat (1) nach einem der Ansprüche 1 bis 4, **dadurch gekennzeichnet, dass** jede Platte (7) der Basis (2) und jede Platte (7) des Anschlussabschnitts (4) über genau zwei Verbindungsstege (6) miteinander verbunden sind.

6. Maxilla-Repositionsimplantat (1) nach einem der Ansprüche 1 bis 3, **dadurch gekennzeichnet, dass** die Verbindungsstege (6) vollständig oder überwiegend parallel zueinander verlaufen.

7. Maxilla-Repositionsimplantat (1) nach einem der Ansprüche 1 bis 6, **dadurch gekennzeichnet, dass** jeder Verbindungssteg (6) ein erstes Ende (20) und ein zweites Ende (21) besitzt, wobei das erste Ende (20) einmaterialig in den Rand (11) eines ersten Schraubenloches (10) der basisseitigen Platte (7) übergeht und das zweite Ende (21) einmaterialig in den Rand (11) eines ersten Schraubenloches (10) der anschlussabschnittseitigen Platte (7) übergeht.

8. Maxilla-Repositionsimplantat (1) nach einem der Ansprüche 1 bis 7, **dadurch gekennzeichnet, dass** die basisseitige Platte (7) über einen ersten Verbindungssteg (6) und einen zweiten Verbindungssteg (6) mit der anschlussseitigen Platte (7) verbunden ist, wobei der erste Verbindungssteg (6) und der zweite Verbindungssteg (6) mit ihren ersten Enden (20) an dem Rand (11) eines einzigen ersten Schraubenloches (10) der basisseitigen Platte (7) angreifen und a) entweder mit ihren zweiten Enden (21) gemeinsam an dem Rand (11) eines einzigen ersten Schraubenloches (10) der anschlussabschnittseitigen Platte (7) angreifen oder b) der eine Verbindungssteg (6) mit seinem zweiten Ende (21) an dem Rand (11) des ersten Schraubenlochs (10) der anschlussabschnittseitigen Platte (7) angreift und der andere Verbindungssteg (6) an dem Rand (11) eines zweiten Schraubenlochs (10) der anschlussabschnittseitigen Platte (7) angreift.

9. Maxilla-Repositionsimplantat (1) nach Anspruch 8, **dadurch gekennzeichnet, dass** der zweite Verbindungssteg (6) sich über die Länge von der basisseitigen Platte (7) aus gesehen zunehmend von dem ersten Verbindungssteg (6) entfernt.

10. Maxilla-Repositionsimplantat (1) nach einem der Ansprüche 1 bis 9, **dadurch gekennzeichnet, dass** jede Platte (7) genau zwei oder mehr Schraubenlöcher (10) besitzt.

## Claims

1. A maxilla repositioning implant (1) for three-dimensional precise orienting of a maxilla displacing portion (5) relative to a remaining maxilla portion (3) fixed to the skull, wherein the maxilla repositioning implant (1) has a base (3) which has at least one plate (7) in which screw holes (10) are provided, wherein a portion of the material of the plate (7) as a rim (11) defines a respective screw hole (10) which is provided for receiving a bone screw (17) in order to ensure fastening to the remaining maxilla portion (3) fixed to the skull via the bone screw (17) to be inserted into the respective screw hole(10), wherein the maxilla repositioning implant (1) furthermore has a connection portion (4) which is spaced from the base (2) via connecting bridges (6), wherein the connection portion (4) has at least one plate (7) in which screw holes (10) are present and wherein a portion of the material of this plate (7) as a rim (11) defines a respective screw hole (10) in order to ensure fastening to the maxilla displacing portion (5) via a bone screw (17) to be inserted into the respective screw hole (10), **characterized in that** two connecting bridges (6) extend from the plate (7) of the connection portion (4) towards the rim (11) of a screw hole (10) in the plate (7) of the base (2).

2. The maxilla repositioning implant (1) according to claim 1, **characterized in that** each of the connecting bridges (6) has an elliptical cross-section.

3. The maxilla repositioning implant (1) according to claim 2, **characterized in that** the elliptical cross-section assumes the specific shape of a circle.

4. The maxilla repositioning implant (1) according to any of claims 1 to 3, **characterized in that** the plate (7) of the base (2) and/or the plate (7) of the connection portion (4) has/have at least one point which has/have a greater thickness than the connecting bridge (6).

5. The maxilla repositioning implant (1) according to any of claims 1 to 4, **characterized in that** each plate (7) of the base (2) and each plate (7) of the connection portion (4) are connected to each other via exactly two connecting bridges (6).

6. The maxilla repositioning implant (1) according to any of claims 1 to 3, **characterized in that** the connecting bridges (6) run completely or mainly parallel to each other.

7. The maxilla repositioning implant (1) according to any of claims 1 to 6, **characterized in that** each connecting bridge (6) has a first end (20) and a second end (21), wherein the first end (20) transitions as a single piece of material into the rim (11) of a first screw hole (10) of the base-side plate (7) and the second end (21) transitions as a single piece of material into the rim (11) of a first screw hole (10) of the plate (7) on the connection portion side.

8. The maxilla repositioning implant (1) according to any of claims 1 to 7, **characterized in that** the base-side plate (7) is connected to the connection-side plate (7) via a first connecting bridge (6) and a second connecting bridge (6), wherein the first connecting bridge (6) and the second connecting bridge (6) engage with their first ends (20) on the rim (11) of a single first screw hole (10) of the base-side plate (7) and a) either engage with their second ends (21) together at the rim (11) of a single first screw hole (10) of the plate (7) on the connection portion side, or b) the one connecting bridge (6) engages with its second end (21) at the rim (11) of the first screw hole (10) of the plate (7) on the connection portion side and the other connecting bridge (6) engages at the rim (11) of a second screw hole (10) of the plate (7) on the connection portion side.

9. The maxilla repositioning implant (1) according to claim 8, **characterized in that** the second connecting bridge (6) progressively increases in distance away from the first connecting bridge (6) over the length as seen from the base-side plate (7).

10. The maxilla repositioning implant (1) according to any of claims 1 to 9, **characterized in that** each plate (7) has exactly two or more screw holes (10).

## Revendications

1. Implant de repositionnement de maxillaire (1) pour l'orientation exacte en trois dimensions d'une section de déplacement de maxillaire (5) par rapport à une section restante de maxillaire (3) solidaire du crâne, dans lequel l'implant de repositionnement de maxillaire (1) possède une base (3) qui présente au moins une plaque (7), dans laquelle des trous de vis (10) sont présents, dans lequel une section du matériau de la plaque (7) comme bord (11) définit respectivement un trou de vis qui est prévu pour la réception d'une vis à os (17) afin d'assurer par l'intermédiaire de la vis à os (17) à insérer dans le trou de vis (10) respectif une fixation à la section restante de maxillaire (3), dans lequel l'implant de repositionnement de maxillaire (1) possède en outre une section de raccordement (4) qui est espacée de la base (2) par l'intermédiaire de nervures de liaison (6), dans lequel la section de raccordement (4) présente au moins une plaque (7), dans laquelle des trous de vis (10) sont présents et dans lequel une section du matériau de cette plaque (7) comme bord (11) définit respectivement un trou de vis (10) afin d'assurer par l'intermédiaire d'une vis à os (17) à insérer dans le trou de vis (10) respectif une fixation à la section de déplacement de maxillaire (5), **caractérisé en ce que** deux nervures de liaison (6) s'étendent vers le bord (11) d'un trou de vis (10) dans la plaque (7) de la base (2) depuis la plaque (7) de la section de raccordement (4).

2. Implant de repositionnement de maxillaire (1) selon la revendication 1, **caractérisé en ce que** chacune des nervures de liaison (6) possède une section transversale elliptique.

3. Implant de repositionnement de maxillaire (1) selon la revendication 2, **caractérisé en ce que** la section transversale elliptique prend la forme spéciale d'un cercle.

4. Implant de repositionnement de maxillaire (1) selon l'une quelconque des revendications 1 à 3, **caractérisé en ce que** la plaque (7) de la base (2) et/ou la plaque (7) de la section de raccordement (4) possède au moins un point qui présente une épaisseur supérieure à celle de la nervure de liaison (6).

5. Implant de repositionnement de maxillaire (1) selon l'une quelconque des revendications 1 à 4, **caractérisé en ce que** chaque plaque (7) de la base (2) et chaque plaque (7) de la section de raccordement (4) sont reliées l'une à l'autre par l'intermédiaire précisément de deux nervures de liaison (6).

6. Implant de repositionnement de maxillaire (1) selon l'une quelconque des revendications 1 à 3, **caractérisé en ce que** les nervures de liaison (6) s'étendent complètement ou principalement parallèlement l'une à l'autre.

7. Implant de repositionnement de maxillaire (1) selon l'une quelconque des revendications 1 à 6, **caractérisé en ce que** chaque nervure de liaison (6) possède une première extrémité (20) et une seconde extrémité (21), dans lequel la première extrémité (20) passe en un matériau dans le bord (11) d'un premier trou de vis (10) de la plaque (7) côté base et la seconde extrémité (21) passe en un matériau dans le bord (11) d'un premier trou de vis (10) de la plaque (7) côté section de raccordement.

8. Implant de repositionnement de maxillaire (1) selon l'une quelconque des revendications 1 à 7, **caractérisé en ce que** la plaque (7) côté base est reliée par l'intermédiaire d'une première nervure de liaison (6) et d'une seconde nervure de liaison (6) à la plaque (7) côté raccordement, dans lequel la première nervure de liaison (6) et la seconde nervure de liaison (6) agissent avec leurs premières extrémités (20) sur le bord (11) d'un seul premier trou de vis (10) de la plaque (7) côté base et a) soit agissent avec leurs secondes extrémités (21) ensemble sur le bord (11) d'un seul premier trou de vis (10) de la plaque (7) côté section de raccordement, soit b) l'une nervure de liaison (6) agit avec sa seconde extrémité (21) sur le bord (11) du premier trou de vis (10) de la plaque (7) côté section de raccordement et l'autre nervure de liaison (6) agit sur le bord (11) d'un second trou de vis (10) de la plaque (7) côté section de raccordement.

9. Implant de repositionnement de maxillaire (1) selon la revendication 8, **caractérisé en ce que** la seconde nervure de liaison (6) s'éloigne progressivement de la première nervure de liaison (6) vu sur la longueur depuis la plaque (7) côté base.

10. Implant de repositionnement de maxillaire (1) selon l'une quelconque des revendications 1 à 9, **caractérisé en ce que** chaque plaque (7) possède précisément deux trous de vis (10) ou plus.
